# EUROPEAN PATENT SPECIFICATION

(11) **EP 1 322 275 B1**
(45) Date of publication and mention of the grant of the patent: **09.01.2013**
(21) Application number: 01973544.8
(22) Date of filing: 25.09.2001
(51) Int. Cl.: A61H 1/00, A61M 11/00

(54) **ULTRASONIC METHOD AND DEVICE FOR WOUND TREATMENT**
ULTRASCHALLVERFAHREN UND EINE VORRICHTUNG ZUR WUNDBEHANDLUNG
PROCEDE ET DISPOSITIF A ULTRA-SONS UTILISES DANS LE TRAITEMENT DES LESIONS

(30) Priority: 25.09.2000 US 669312
(43) Date of publication of application: 02.07.2003
(73) Proprietor: Celleration, Inc., Eden Prairie, MN 55346 (US)
(72) Inventor: BABAEV, Eliaz, Minnetonka, MN 55345 (US)
(74) Representative: Grey, Ian Michael
(86) International application number: PCT/US2001/030096
(87) International publication number: WO 2002/024150

(56) References cited:
- EP-A- 0 416 106
- WO-A-97/17933
- US-A- 4 767 402
- US-A- 5 315 998
- US-A- 5 551 416
- ZHAROV VLADIMIR P ET AL: "Comparison possibilities of ultrasound and its combination with laser in surgery and therapy" May 2000 (2000-05), PROC SPIE INT SOC OPT ENG; PROCEEDINGS OF SPIE - THE INTERNATIONAL SOCIETY FOR OPTICAL ENGINEERING 2000 SOCIETY OF PHOTO-OPTICAL INSTRUMENTATION ENGINEERS, BELLINGHAM, WA, USA, VOL. 3916, PAGE(S) 331 - 339 , XP002294505 * page 333, paragraph 4 - page 334, paragraph 1; figure 1b *
- DATABASE WPI Section Ch, Week 198509 Derwent Publications Ltd., London, GB; Class B07, AN 1985-055284 XP002294506 -& SU 1 106 485 A (MOSCOW BAUMAN TECH COLL) 7 August 1984 (1984-08-07)
- DATABASE WPI Section PQ, Week 199501 Derwent Publications Ltd., London, GB; Class P33, AN 1995-004946 XP002294507 -& SU 1 827 239 A1 (OMSK POLY) 15 July 1993 (1993-07-15)
- DATABASE WPI Section Ch, Week 198304, Derwent Publications Ltd., London, GB; Class A32, AN 1983-09189K, XP002294548 & SU 914 099 A (MICROCLIMAZE PLAN) 23 March 1982

## Description

Ultrasonic waves has been widely used in medical applications, including both diagnostics and therapy as well as many industrial applications. One diagnostic use of ultrasound waves includes using ultrasonic waves to detect underlying structures in an object or a human tissue. In this procedure, an ultrasonic transducer is placed in contact with the object or tissue via a coupling medium and high frequency (1-10 MHz) ultrasonic waves are directed into the tissue. Upon contact with various underlying structures, the waves are reflected back to a receiver adjacent the transducer. By comparison of the signals of the Ultrasonic wave as sent with the reflected ultrasonic wave as received, an image of the underlying structure can be produced. This technique is particularly useful for identifying boundaries between components of tissue and can be used to detect irregular masses, tumors, and the like.

Two therapeutic medical uses of ultrasound waves include aerosol mist production and contact physiotherapy. Aerosol mist production makes use of a nebulizer or inhaler to product an aerosol mist for creating a humid environment and delivering drugs to the lungs. Ultrasonic nebulizers operate by the passage of ultrasound waves of sufficient intensity through a liquid, the waves being directed at an air-liquid interface of the liquid at a point underneath or within the liquid. Liquid particles are ejected from the surface of the liquid into the surrounding air following die disintegration of capillary waves produced by the ultrasound. This technique can produce a very fine dense fog or mist. Aerosol mists produced by ultrasound are preferred over aerosol mists produced by other methods because a smaller particle size of aerosol can be obtained with the ultrasonic waves. One of the major shortcomings of inhalers and nebulizers is that the aerosol mist cannot be directed to a target area without an air stream, which decreases the efficiency of ultrasound.

Ultrasonic sprayers such as those sold by Sonic and materials Inc., Misonix Inc., Sono-Tek Inc. (see, for example, U. S. Patents Nos. 4,153,201,4,655,393, and 5,516,043) operate by passing liquid through a central orifice of an ultrasound instrument-tip. Major disadvantages of these sprayers include non-uniform particle size, heating of liquid flow, and less efficiency of ultrasound waves because of demolished end (radiation) surface of tip.

Contact physiotherapy applies ultrasonic waves directly to tissue in an attempt to produce a physical change in the tissue. In conventional ultrasound physiotherapy, an ultrasonic wave contacts the tissue via a coupling medium. Ultrasonic waves produced by the transducer travel through the coupling medium and into the tissue. The coupling medium is typically a bath of liquid, a jelly applied to the surface to be treated, or a water-filled balloon. Conventional techniques provide ultrasonic waves having an intensity of about 0.25 w/cm² to 3 w/cm² at a frequency of about 0.8 to 3 Megahertz. The treatment is applied to a skin surface for from about 1 to 30 minutes, two or three times a week. The coupling medium can provide a cooling effect which dissipates some of the energy produced by the ultrasonic transducer.

More importantly, a coupling medium or direct contact between the tissue and ultrasonic transducer is necessary to transmit the ultrasonic waves to the skin surface because ambient air is relatively poor medium the propagation of ultrasonic waves.

Several beneficial effects have been reported from contact ultrasound physiotherapy, such as, for example, the following: local improvement of the blood circulation, heating of the tissue, accelerated enzyme activity, muscle relaxation, paid reduction, and enhancement of natural healing processes. Despite these beneficial effects, current techniques of medical physiotherapy using ultrasonic waves are limited by the necessity of providing a direct contact interface between the ultrasonic transducer and the tissue to maintain an effective transmission of the ultrasonic waves from the transducer to the tissue.

The necessity of direct contact with or without a coupling medium makes current methods undesirable. Some tissue conditions may be accessible to contact ultrasound devices but would be impractical for contact ultrasound treatment. For example, fresh or open wounds resulting from trauma, burns, surgical interventions are not suitable for direct contact ultrasound treatment because of the structural nature of the open wound and the painful condition associated with those wounds. Moreover, conventional contact ultrasound may have a destructive effect on these types of open wounds due to the close proximity of an oscillating tip of an ultrasonic transducer relative to the already damaged tissue surface.

It is also known from WO97/17933A to provide an apparatus for use in treating a wound comprising means for generating ultrasonic energy positioned at a non-contact distance from the surface of the wound, a reservoir containing a liquid and means for delivering the liquid to a lateral or distal surface of an ultrasonic transducer tip to produce a spray, wherein the generated ultrasonic energy is delivered to the wound through a spray, and wherein the ultrasonic energy penetrates the wound tissue.

Apparatus for use in treating a wound according to the invention is characterised in that the liquid contained in the reservoir is saline solution.

Embodiments of the invention will now be described, by way of example only, with reference to the accompanying drawings, in which:
Figure 1 is a perspective view of a ultrasonic wound treatment system for use according to the present invention;
Figure 2 is a lateral schematic view of an ultrasonic sprayer useful according to the present invention;
Fig. 3 is a partly cross-sectional view of an ultrasonic sprayer in use according to the present invention;
Figs. 4a and 4b are each a detail of the sprayer shown in Fig. 3 for spraying liquid from a radiation surface (FIG. 4a) or from a side (radial) surface, based on the Babaev effect (FIG. 4b);
Fig. 5 represents a cross-sectional view of the distal end of the ultrasonic transducer when liquid is delivered to the side or radiation surface of the transducer tip from 360° by perimeter as a top, side, bottom, etc;
Fig. 6 is a variation of the detail of Fig. 4b which illustrates the spraying effect by changing the angle between the ultrasound instrument and horizontal line from 0° to 90°;
Figs. 7a to 7g are each a cross-sectional view of a useful ultrasound tip;
Figs. 8a to 8i are each an enlarged view of a different modification of a tip end shape of the ultrasonic sprayer used according to the present invention; and
Figs. 9a, 9b, and 9c represent cross-sectional, distal, and lateral views of the top of an ultrasonic sprayer having a slot, groove, or thread.

### DETAILED DESCRIPTION OF THE INVENTION

The device of the invention that produces a spray is characterized by means for first delivering the liquid to a lateral surface of an ultrasonic transducer tip adjacent to a free end surface such that the liquid is pulled to the free end surface by a vacuum (negative pressure) created by the ultrasound waves on the free end surface of the transducer tip. This effect can be achieved while the angle between the ultrasound instrument and the horizontal is modified up to 90°. (This acoustical effect of delivering liquid from radial side of a tip to the free end was discovered by the inventor of this invention and is called the "Babaev effect".) This effect occurs when liquid is delivered to the radial surface of a transducer tip from 360° by perimeter as a top, side, bottom, etc. For the above purpose the device must have a so-called nozzle from steel (non- disposable) or plastic (disposable) with a different design of valve. The nozzle allows delivery of liquid to the lateral surface of the transducer tip or directly to the distal side (radiation surface) of the ultrasound transducer to act as a sprayer or atomizer.

One of the major advantages of the apparatus of the invention is die uniformity of the spray particles generated. Because liquid is sprayed from a solid radiation surface, there is substantial uniformity of particle size, about 90% or greater, preferably from about 90 to 96%.

The step of producing the spray using the apparatus of the invention can further include operating the transducer to produce ultrasonic waves having a frequency of from about 18kHz to 10,000 MHz. Frequencies below 18 kHz, i. c., from about 1 to 18 kHz, can be used as well; however, this lower range is less desirable because this range of sound wave can be uncomfortable to the patient and operator (without ear protection or the like). Frequencies in the range of from about 30 to 100 kHz are preferred, and frequencies of about 40 kHz are most preferred.

The separation distance between the free end surface of the transducer and the surface or object to be sprayed should be a "non-contact" distance of at least 0.1 in. (2.5 mm). Preferably the separation distance is from about 0.1 in. (2.5 mm) to 20 in. (51 cm), more preferably from about 0.1 in. (2.5 mm) to 5 in. (12.7 cm). The liquid to be sprayed can be any appropriate carrier such as water (regular or distilled), saline solution, or oil to be applied to tissue, such as a vegetable, peanut, or canola oil, optionally with a soluble pharmaceutical (e. g., an antibiotic), antiseptic, conditioner, surfactant, emollient, or other active ingredient. The pharmaceutical or the like is preferably present in a concentration sufficiently low to be soluble but high enough to be effective for the intended purpose.

It is withing the scope of the invention that the liquid to be sprayed using the apparatus could comprise a mixture of two or more immiscible liquids, at least one of which is saline solution or a heterogeneous mixture of saline solution and small particles.

The spray produced using the apparatus according to the invention is directed to the object, surface, or tissue to be sprayed for the time and frequency required to accomplish a particular purpose or treatment. It is believed that a minimum length of spray of at least one second will be required; however, the length or duration of the spray could be from about one second to as much as a minute or more, even 30 minutes. Numerous factors or circumstances, such as, for example, the area to be sprayed (e. g., the size of a wound), the volume rate of spray produced, the concentration of active ingredient, etc., will impact upon the duration and/or frequency of the spraying. Spraying could be required from one ore more times daily to as little as two or three times a week or month.

Ultrasonic waves are applied to a wound without establishing contact, directly or indirectly, between the ultrasonic transducer and the wound. For example, surfaces of the human body especially suited for treatment with the method of the present invention include infected and inflammatory situations in open wounds, including trauma or gun shut wounds, fire and chemical burns.

In addition, the apparatus of the present invention is particularly suited to directing a spray into orifices or other body crevices that are difficult to access.

Wound treatment using the apparatus according to die invention has several advantages. First, it topically applies medicines such as liquid antibiotics to the wound surface without the need to contact infected, inflamed or painful tissue with an instrument. And second, a significant bactericidal effect occurs when a wound surface is sprayed using the apparatus of the present invention.

Moreover, aside from the bactericidal effect and advantages of non-contact treatment, using the apparatus of the present invention gave a significant reduction in volume used of liquid medicine used as compared with traditional methods for wound treatment. Similarly, this allows for precise dosage of the sprayed liquid to permit a user, such as a physician, to administer the desired volume of liquid at a desired rate and duration.

It has been found that use of the apparatus of the present invention decreases heating times for inflammatory and purulent infected wounds that is from about 1.5 to 3 times faster than traditional methods. This effect results from a bactericidal, blood flow increasing and mechanical cleansing effect of the atomized spray particles, which have ultrasound energy due to the ultrasonic waves. The spray mechanically scrubs the surface of tissue to remove dirt, dead tissue, and purulent buildup on the tissue surface. The mentioned healing effect also results of energized and highly activated antibiotics, drug penetration into the tissue surface up to 0.5 mm in depth under influence of ultrasound waves.

Additionally, a combination of the low frequency ultrasonic waves and the sonicated medicines (highly activated by ultrasonic energy) destroy the surface bacteria to result in a higher disinfecting property of sonicated liquids as compared to ordinarily applied liquids. The spray of the present method also stimulates healthy cell growth to aid in granulization and epithelization of the healing tissue.

The apparatus of the present invention offers an approach that may re-establish use of some traditional antibiotics and establish a method fighting bacteria without antibiotics when necessary. The effect of the method of the present invention in highly activating antibiotics may allow some traditional antibiotics to overcome bacteria which have become resistant to that antibiotic. Moreover, independent of the sonication effect of the antibiotics, the low frequency ultrasonic waves applied in the method of the present invention physically destroy bacteria. The combination of the highly activated antibiotics and of the low frequency ultrasonic waves in which the saline solution includes an antibiotic, produces a strong bactericidal effect not found in mere topically application or orally ingested antibiotic. This combined effect has been shown to significantly increase the healing of purulent infected wounds.

The apparatus also provides a system of non-contact drug delivery without use of a compression sprayer system. This simplifies the design of a non-contact drug delivery sprayer and reduces the weight of the sprayer. More importantly, not using compression to propel the atomized particles preserves the ultrasound energy carried by the spray particles. This ultrasound energy has been proven to destroy bacteria by action of the ultrasonic waves and, optionally, by highly activated liquid medicines applied to the tissue.

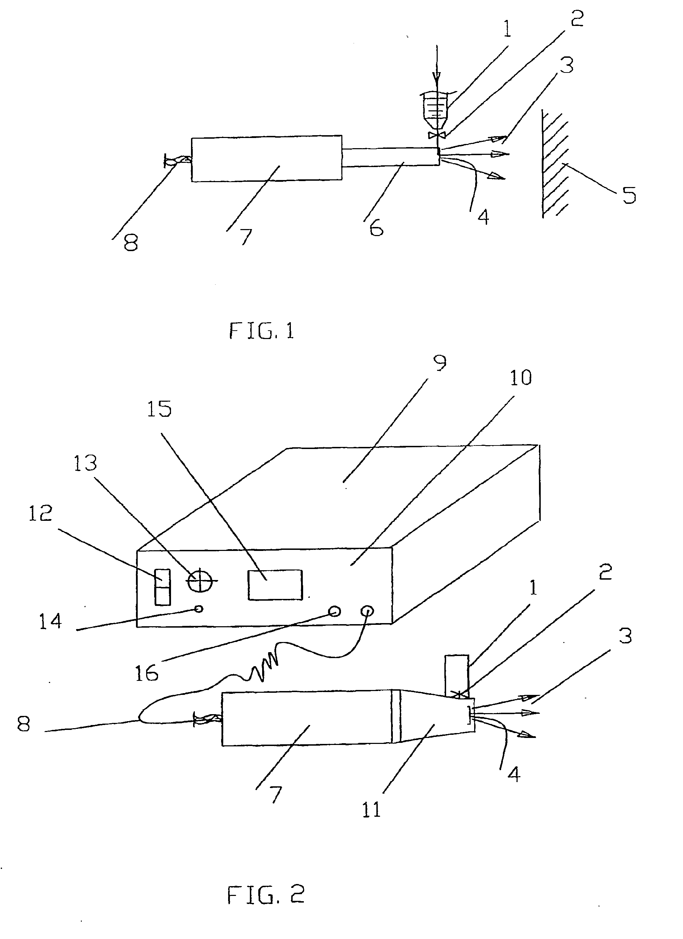

The invention can perhaps be better appreciated by making reference to the drawings. In Figure 1, an ultrasonic treatment system 2 comprises an ultrasound wave generator 4, connected to an ultrasound transducer 6 by a cable 8. The wave generator 4, which is conventional, may have a front panel 10 with a power button 12, a timer 14, a control button 16, one or more displays 18, and one or more jacks 20, for example, for a footswitch (not shown). A nozzle 22 having a liquid reservoir 24 with a valve 26 is attached to the distal portion of transducer 6. Arrows 28 represent the direction of spray produced.

Figure 2 is a simplified representation of an ultrasonic device and spray according to the invention. Transducer 6 has a distal transducer tip or horn 30. Liquid from a liquid reservoir 32 flows through a valve 34 to a position adjacent the distal radiation surface 36 of a horn 30. Transducer 6 is attached to an ultrasound source via cable 8. A liquid mist is directed in the direction of arrows 38 to target tissue or surface 40.

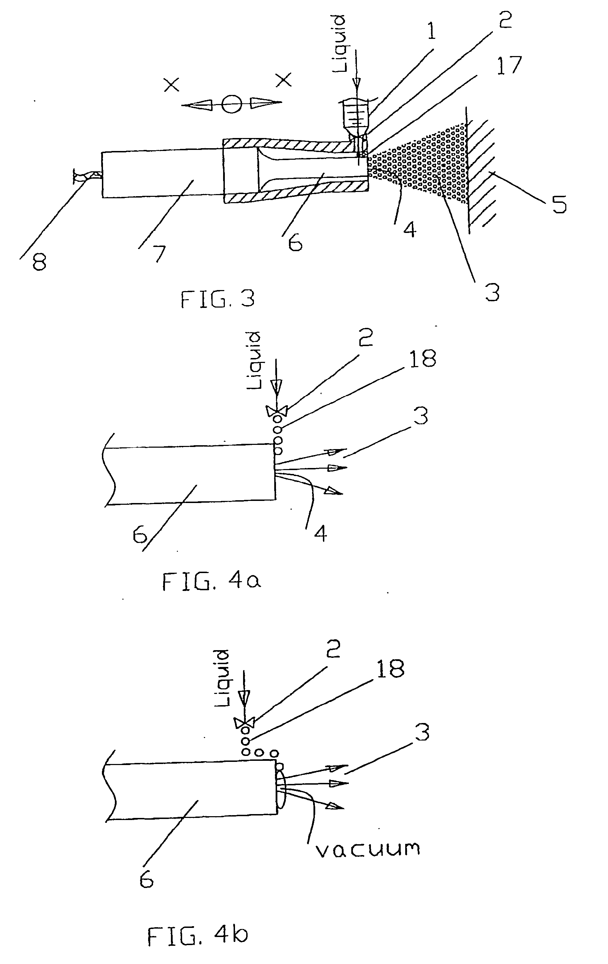

Figure 3 is an enlarged, partly cross-sectional view of a section of Figure 1 illustrating a spray created by the device according to the present invention. This device is a modification and implementation of a device disclosed in US Patent No. 5076266, which is incorporated herein by reference. As can be seen in more detail in Figure 3, nozzle 22 surrounds ultrasound horn 30. Also, liquid reservoir 32 has a valve 34 positioned between reservoir 32 and the distal surface 36 of ultrasonic horn 30. A conical spray pattern of liquid droplets 42 is directed at a surface or tissue 44 of a target. This configuration is effective to spray liquid onto a surface and to deliver ultrasonic waves to that surface, such as, for example, the surface of a wound.

Valve 34 allows liquid to flow to distal tip 36 as drops or continuous flow through gap 46. Valve 34 may be located anywhere, including between reservoir 32 and horn 30. Mechanical movement of the horn 30 in the direction x-x causes liquid to flow to the distal end or radial surface 36.

Figure 4a is a view of the ultrasonic sprayer as used in the present invention for sparying liquid 48 directed to distal end (radiation surface) 36.

Figure 4b is a view of the basis spraying method from side (radial) surface of the tip based on the Babaev effect. In this case liquid or drug directed to the radial surface 36 of ultrasound horm 30 becomes sonicated (ultrasonically engergixed), after being pulled forward by negative pressure (vacuum) created by ultrasound waves and sprays.

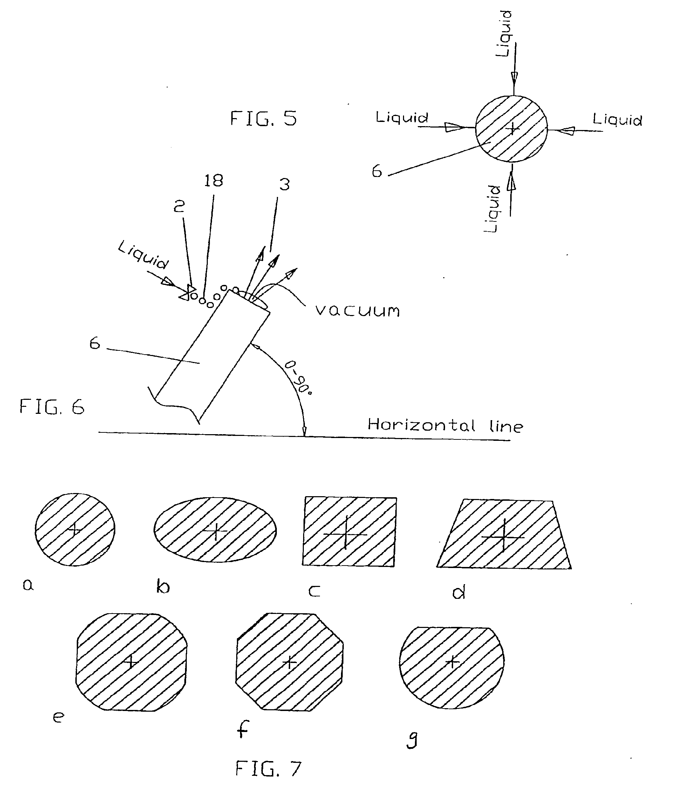

As shown in Figure 5, liquid is deliered to the side or radiation surface 36 of transducer horn 30 and 360° along its perimeter as a top, side, bottom etc.

In the embodiment of the invention shown in Figure 6, a partial section of transducer horn 30 is elevated from the horizontal up to 90°. Due to the Babaev effect, liquid 48 is still travels to radiation surface 36.

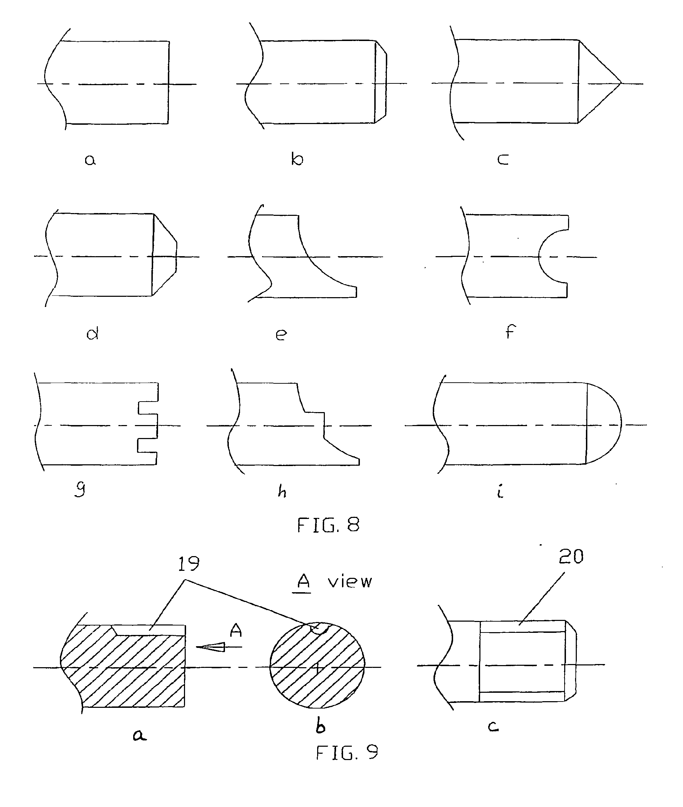

The ultrasound tip or horn can have a regular or irregular lateral cross-section, including circular, oval, elliptical, rectangular, trapezoidal, or a combination thereof. See, for example, Figures 7a to 7g, which are each a view of a cross-section of a ultrasound tip or horn. Also, the distal end shape of the ultrasound tip or horn longitudinal cross-section may vary and may be rectangular, elliptical, oval, spherical, conical, curved, stepped, with chamfer, etc. See for example, Figures 8a to 8i, which are each an enlarged view in section of a different modification of a tip of the sprayer as used in the apparatus of the present invention. The preferred shape is rectangular, because of radiation beams from ultrasound tip surface fully directed to the target (wound). With the spheric, elliptic and oval (Fig. 8e) form of shape end radiation beams are focused at focal point With other form of shape end, radiation beams are getting spreaded reaching target partly.

Radial side surface of distal end of the tip may have slot (groove) or thread for liquid to be directed to the radiation surface (Figs. 9a to 9c).

Figs. 9a to 9c are each a view of radial side surface of distal end of the tip which has a slot (groove) 19 or thread 20 for liquid to be directed to the radiation surface.

The preceding specific embodiments are illustrative of the practice of the invention It is to be understood, however, that other expedients known to those skilled in the art or disclosed herein, may be employed without departing from the spirit of the invention or the scope of the appended claims.

## Claims

1. An apparatus for use in treating a wound comprising:
means for generating ultrasonic energy positioned at a non-contact distance from the surface of the wound;
a reservoir (24,32) containing a liquid; and
means for delivering the liquid to a lateral or distal surface of an ultrasonic transducer tip to produce a spray, wherein the generated ultrasonic energy is delivered to the wound through the spray, and wherein the ultrasonic energy penetrates the wound tissue,
**characterised in that** the liquid contained in the reservoir (24,32) is saline solution.

2. An apparatus according to claim 1, wherein the means for generating ultrasonic energy generates ultrasonic waves having a frequency from 18 kHz to 10,000 MHz.

3. An apparatus according to claim 2, wherein the means for generating ultrasonic energy generates ultrasonic waves having a frequency from 30 kHz to 100 kHz.

4. An apparatus according to claim 2, wherein the means for generating ultrasonic energy generates ultrasonic waves having a frequency of about 40 kHz.

5. An apparatus according to claim 2, further comprising a plastic disposable nozzle.

## Patentansprüche

1. Vorrichtung zur Verwendung bei der Behandlung einer Wunde, umfassend:
eine Einrichtung zum Erzeugen von Ultraschallenergie, die in einem kontaktfreien Abstand zur Oberflache der Wunde positioniert ist;
ein Reservoir (24, 32), das eine Flüssigkeit enthält; und
eine Einrichtung zum Zuführen der Flüssigkeit zu einer lateralen oder distalen Oberfläche einer Ultraschallschwingerspitze, um ein Spray zu erzeugen, wobei die erzeugte Ultraschallenergie der Wunde über das Spray zugefuhrt wird, und wobei die Ultraschallenergie das Wundgewebe durchdringt,
**dadurch gekennzeichnet, dass** die Flussigkeit, die in dem Reservoir (24, 32) enthalten ist, eine Salzlösung ist.

2. Vorrichtung nach Anspruch 1, wobei die Einrichtung zum Erzeugen von Ultraschallenergie Ultraschallwellen mit einer Frequenz von 18 kHz bis 10.000 MHz erzeugt.

3. Vorrichtung nach Anspruch 2, wobei die Einrichtung zum Erzeugen von Ultraschallenergie Ultraschallwellen mit einer Frequenz von 30 kHz bis 100 kHz erzeugt.

4. Vorrichtung nach Anspruch 2, wobei die Einrichtung zum Erzeugen von Ultraschallenergie Ultraschallwellen mit einer Frequenz von ungefähr 40 kHz erzeugt.

5. Vorrichtung nach Anspruch 2, ferner umfassend eine Einweg-Kunststoffdüse.

## Revendications

1. Appareil pour une utilisation dans le traitement d'une plaie, comprenant :
un moyen pour générer de l'énergie ultrasonique positionné à une distance sans contact de la surface de la plaie ;
un réservoir (24, 32) contenant un liquide ; et
un moyen pour délivrer le liquide à une surface latérale ou distale d'un embout de transducteur ultrasonique pour produire un liquide d'atomisation, dans lequel l'énergie ultrasonique générée est délivrée à la plaie par liquide d'atomisation, et dans lequel l'énergie ultrasonique pénètre dans le tissu de la plaie,
**caractérisé en ce que** le liquide présent dans le réservoir (24, 32) est une solution saline.

2. Appareil suivant la revendication 1, dans lequel le moyen pour générer de l'énergie ultrasonique génère des ondes ultrasoniques ayant une fréquence de 18 kHz à 10 000 MHz.

3. Appareil suivant la revendication 2, dans lequel le moyen pour générer de l'énergie ultrasonique génère des ondes ultrasoniques ayant une fréquence de 30 kHz à 100 kHz.

4. Appareil suivant la revendication 2, dans lequel le moyen pour générer de l'énergie ultrasonique génère des ondes ultrasoniques ayant une fréquence d'environ 40 kHz.

5. Appareil suivant la revendication 2, comprenant en outre une buse jetable en matière plastique.
